(19) **Europäisches Patentamt**

**European Patent Office**

**Office européen des brevets**

(11) **EP 0 902 284 B1**

(12) **FASCICULE DE BREVET EUROPEEN**

(45) Date de publication et mention
de la délivrance du brevet:
**15.10.2003 Bulletin 2003/42**

(51) Int Cl.7: **G01N 33/42**, G01N 33/44,
C08L 95/00

(21) Numéro de dépôt: **98402163.4**

(22) Date de dépôt: **01.09.1998**

(54) **Procédé d'optimisation d'un mélange bitume/polymères**

Verfahren zur Optimierung einer Bitumen-Polymermischung

Method for optimising a bitumen/polymeric mixture

(84) Etats contractants désignés:
**AT BE CH DE DK ES FI FR GB GR IT LI NL PT SE**

(30) Priorité: **12.09.1997 FR 9711406**

(43) Date de publication de la demande:
**17.03.1999 Bulletin 1999/11**

(73) Titulaire: **Onduline**
**75818 Paris Cédex 17 (FR)**

(72) Inventeur: **Ruffenach, François**
**76000 Rouen (FR)**

(74) Mandataire: **Michelet, Alain et al**
**Cabinet Harlé et Phélip**
**7, rue de Madrid**
**75008 Paris (FR)**

(56) Documents cités:
DE-A- 3 908 831        FR-A- 2 733 764
GB-A- 2 244 131        US-A- 4 357 169
US-A- 5 326 798        US-A- 5 365 793
US-A- 5 659 140

• LEPENIOTIS S S, VIGEZZI M J: "Lowering
manufacturing cost of material by formulating it
through statistical modeling and design",
CHEMOMETRICS AND INTELLIGENT
LABORATORY SYSTEMS, , , Vol. 29, no. 1, pages
133 à 139
• BABCOCK L M, ALTEKAR M: "Use of statistical
design to develop flame retardant polymer
formulations", CHEMOMETRICS AND
INTELLIGENT LABORATORY SYSTEMS, , , Vol.
29, no. 1, pages 141 à 146

**Description**

L'invention est relative à la fabrication de mélanges bitume/polymères, notamment destinés à la production de membranes d'étanchéité pour toitures.

Les polymères utilisés dans ces mélanges peuvent être de natures diverses. On utilise classiquement des polymères recyclés ou sous forme de déchets, pour réduire le coût des matières premières.

On peut notamment citer l'APP (polypropylène atactique) qui constitue un déchet de fabrication de l'IPP (polypropylène isotactique).

Les caractéristiques physiques de ces polymères recyclés ne sont pas constantes. Or les fabricants de membranes d'étanchéité doivent respecter des spécifications techniques et garantir la qualité de leurs produits.

Les fabricants ne disposent que de moyens empiriques pour prévoir les caractéristiques physiques d'un mélange bitume/polymères, lorsque l'on fait varier la quantité des polymères ou lorsque la nature des polymères est modifiée.

Pour chaque nouveau polymère ou pour chaque nouvelle livraison d'un polymère recyclé ou sous forme de déchets, les fabricants doivent réaliser des essais pour ajuster la quantité de polymères dans le mélange. De plus, en pratique, les polymères sont utilisés en excès pour garantir la qualité du produit final, ce qui augmente les coûts. Enfin, des contrôles de qualité fréquents et coûteux sont nécessaires.

Lorsqu'un contrôle de qualité fait apparaître que les caractéristiques du mélange bitume/polymères obtenu ne correspondent pas aux spécifications techniques, ce sont également des moyens empiriques qui sont mis en oeuvre pour corriger le mélange, notamment en y incorporant des quantités supplémentaires de polymères.

Dans le domaine technique des membranes d'étanchéité, relativement ancien, ces moyens empiriques de détermination des mélanges bitume/polymères sont les seuls à être utilisés. Le nombre d'essais est nécessairement limité pour ne pas bloquer la production.

La mise en oeuvre de méthodes plus rationnelles est, de façon générale, rejetée, compte-tenu des risques de ralentissement de la fabrication.

La modification des caractéristiques d'un mélange par la variation des pourcentages des différentes composantes de ce mélange est connue, par exemple, du document US-A-5326798. Ce document divulgue toute une série d'exemples de compositions avec différents rapports bitume/polymères ainsi que les caractéristiques physiques résultantes. Ces mélanges bitume/polymères sont utilisés pour la réalisation de membranes étanches.

Des méthodes statistiques pour optimiser la composition d'un mélange sont connues de deux documents, à savoir une publication de LEPENIOTIS et VIGEZZI dans Chemometrics and Intelligent Laboratory Systems, volume 29, N°1 ayant pour titre "Lowering manufacturing cost of material by formulating it through statistical modeling and design" et une publication de BABCOCK et ALTEKAR dans la même revue que la publication précédente et ayant pour titre "Use of statistical design to develop flame retardant polymer formulations". Le premier de ces deux documents décrit l'utilisation de différentes méthodes statistiques pour déterminer certaines caractéristiques d'un matériau à améliorer. Le second de ces deux documents décrit plus précisément l'utilisation de deux méthodes statistiques pour déterminer le temps de combustion d'un matériau destiné à retarder un feu en fonction de différentes compositions. Les compositions, notamment trois composantes, sont exprimées par des valeurs normalisées -1, 0 et +1 et le temps de combustion est indiqué par une formule.

Toutefois, aucun des trois documents cités ci-avant n'enseigne une méthode ni un procédé permettant de déterminer l'influence de la variation de la composition d'un mélange bitume/polymère sur au moins une caractéristique physique du mélange, à partir de seulement quelques essais supplémentaires.

L'invention a pour objet de pallier ces inconvénients en proposant un procédé pour déterminer l'influence du pourcentage des différents polymères d'un mélange bitume/polymères sur au moins une caractéristique physique du mélange, à partir d'un nombre d'essais limité qui sont effectués une fois pour toutes, pour les polymères et le bitume en cause.

Ainsi, le procédé selon l'invention consiste:

- à réaliser un nombre limité de mélanges bitume/polymères, selon la méthode des plans d'expériences, les polymères et le bitume étant identiques dans tous les mélanges et les pourcentages respectifs des polymères pouvant être fixés à un niveau minimum, d'une gamme de valeurs déterminée à l'avance pour chacun d'eux,
- à mesurer la(les) dites) caractéristique(s) physique(s) pour chacun desdits mélanges,
- et à en déduire, par des calculs statistiques, une modélisation de la (des) dite(s) caractéristique(s) physique(s).

Le procédé selon l'invention présente l'avantage de pouvoir tenir compte de l'influence d'un autre polymère qui serait introduit dans le mélange.

Le procédé consiste alors de plus:

- à réaliser au moins quatre mélanges supplémentaires incorporant un autre polymère,
- à mesurer la(les)dite(s) caractéristique(s) physique(s) pour chacun de ces mélanges supplémentaires,
- et à en déduire les modifications à apporter à la modélisation de la (des)dite(s) caractéristique(s) physique(s), pour tenir compte de l'introduction de cet autre polymère.

Le procédé selon l'invention n'est pas limité à l'introduction d'un seul autre polymère, les étapes précédentes du procédé peuvent être répétées pour tout autre polymère qui devrait être introduit dans le mélange.

Le mélange bitume/polymères peut comporter des charges. Le pourcentage de charges dans le mélange peut être constant.

La(les) caractéristique(s) physique(s) du mélange est(sont) notamment la flexibilité à basse température à l'état neuf et à l'état vieilli, la viscosité Brookfield à 180°C, la pénétration à 25°C et 60°C et/ou la température de ramollissement.

Pour la fabrication de membranes d'étanchéité, les polymères dudit mélange bitume/polymères sont choisis, à titre d'exemple, parmi les APP, IPP et les copolymères du type éthylène/propylène.

L'invention concerne également un procédé pour déterminer au moins une caractéristique physique d'un mélange bitume/polymères en fonction des polymères utilisés.

Ce procédé consiste :

- à mettre en oeuvre le procédé selon l'invention de détermination de l'influence du pourcentage des différents polymères du mélange sur la (les) dite(s) caractéristique(s) physique(s),
- et à fixer les pourcentages de chaque polymère pour en déduire la valeur de la (des) dite(s) caractéristique(s) physique(s).

L'invention concerne également un procédé d'obtention d'un mélange bitume/polymères présentant une (des) caractéristique(s) physique(s) du mélange souhaitée(s).

Le procédé consiste :

- à mettre en oeuvre le procédé selon l'invention de détermination de l'influence du pourcentage des différents polymères du mélange sur la(les) dite(s) caractéristique(s) physique(s),
- à fixer une gamme de valeurs pour la(les)dite(s) caractéristique(s) et
- à déduire le pourcentage de chaque polymère dans le mélange.

Ce procédé consiste à déterminer les pourcentages des polymères en tenant compte de leur coût.

Ces procédés permettent d'optimiser les mélanges bitume/polymères en termes de qualité et de coût de matières premières, tout en étant rapides et peu coûteux à mettre en oeuvre.

L'invention concerne aussi un procédé de modification d'un mélange bitume/polymères présentant une(des) caractéristique(s) physique(s) déterminée(s) à modifier.

Ce procédé consiste :

- à mettre en oeuvre le procédé selon l'invention de détermination de l'influence du pourcentage des différents polymères du mélange sur la (les) dite(s) caractéristique(s) physique(s),
- à fixer d'autres valeurs de la(des)dite(s) caractéristique(s) physique(s) dudit mélange et
- à calculer la quantité supplémentaire d'au moins un polymère à ajouter dans ledit mélange pour qu'il présente des caractéristiques physiques de valeurs sensiblement égale à ces autres valeurs.

Tous ces procédés nécessitent de déterminer au préalable l'influence des différents polymères sur le mélange, mais leur mise en oeuvre ne nécessite aucun essai complémentaire lors de la fabrication.

L'invention sera mieux comprise et d'autres buts, avantages et caractéristiques de celle-ci apparaîtront plus clairement à la lecture de la description détaillée qui suit.

L'optimisation des mélanges bitume/polymères nécessite de connaître l'influence de chacun des constituants du mélange sur les caractéristiques physiques de ce dernier.

Les caractéristiques physiques retenues sont par exemple imposées par des normes ou classiquement choisies par les fabricants.

On peut notamment citer :

- la flexibilité à basse température (exprimée en degrés Celsius) à l'état neuf et à l'état vieilli (après 4 semaines à 80°C), mesurée selon une méthode dite UEATC (Union Européenne des Agréments Techniques de la Construction),

- la viscosité Brookfield à 180°C (exprimée en Pa·s, entre parenthèses en centipoises), mesurée avec un appareil de mesure de viscosité rotatif (aiguille 28, vitesse 50 tours/mn),
- la pénétration à 25°C, et à 60°C (exprimée en deci mm), mesurée selon la norme NFT 66-004,
- la température de ramollissement (exprimée en degrés Celsius), mesurée selon la norme NFT 66-008.

Dans la mesure où les caractéristiques physiques du bitume modifié par les polymères dépendent de la nature de chaque polymère et de son pourcentage dans le mélange, ainsi que du bitume choisi, un nombre considérable d'essais à réaliser est théoriquement nécessaire pour établir des caractéristiques physiques modélisées de façon fiable. Ceci est dissuasif pour l'homme du métier et c'est pourquoi aucun modèle n'a été mis au point dans ce domaine technique.

L'invention utilise une méthode dite des plans d'expériences, qui permet de réduire le nombre de tests à réaliser pour modéliser les caractéristiques physiques du mélange, tout en permettant d'obtenir des modèles fiables. De plus, cette méthode met en évidence les interactions entre les différents polymères.

On peut à cet égard se référer à l'ouvrage de J. GOUPY : « la Méthode des plans d'expériences », paru aux Editions Dunod en octobre 1988.

L'application de cette méthode dans le domaine des mélanges bitume/polymères va maintenant être décrite.

La modélisation des caractéristiques physiques du mélange est établie pour un bitume donné.

Le bitume pris ici pour exemple présente les caractéristiques suivantes :

| | BITUME |
|---|---|
| Température de ramollissement (°C) | 44.0 |
| Pénétration à 25°C (mm/10) | 112 |
| Viscosité cinématique à 135°C (mm²/s) | 290 |

Sa composition est la suivante :

| | *Bitume* |
|---|---|
| asphaltènes | 16.3 |
| résines | 19.9 |
| aromatiques | 55.7 |
| saturés | 8.1 |

Dans cet exemple, le mélange bitume/polymères est chargé avec des charges minérales, dont le pourcentage dans le mélange est d'environ 18%.

Les polymères qui entrent dans la composition d'un bitume modifié ne sont en général pas constitués d'un produit unique.

Ils comprennent souvent différents IPP et APP ainsi que des copolymères.

Dans l'exemple qui est ici décrit, 6 polymères ont été retenus dont les caractéristiques sont les suivantes :

| | |
|---|---|
| IPP | Polymère très dur et résistant dont l'indice de fluidité (Melt Flow Index) est de l'ordre de 8 |
| APP1 | Viscosité Brookfield à 180°C ~ 0,5 Pa·s (500 CP) et pénétration à 25°C ~ 10-20mm/10 |
| APP2 | Viscosité Brookfield à 180°C ~ 3 Pa·s (3000 CP) et pénétration à 25° <10 mm/10 |
| COPO1 | Viscosité Brookfield à 180°C ~ 1000 Pa·s (1 000 000 CP) Bonne flexibilité à basses températures |
| COPO2 | Viscosité Brookfield à 180°C ~ 700 Pa·s (700 000 CP) - Plus dur et moins flexible que COPO1 |
| COPO3 | Viscosité Brookfield à 180°C ~ 800 Pa·s (800 000 CP) - Plus dur et moins flexible que COPO2 |

Le plan d'expériences est basé sur six variables indépendantes (IPP (en %), APP1(en %), APP2(en %), COPO1(en %), COPO2 (en %) et COPO3 (en %)). Ainsi, 64 mélanges doivent être réalisés (le bitume et les charges étant maintenus identiques dans leur nature, le pourcentage de charges étant dans cet exemple maintenu constant et le pourcentage de bitume étant le complément à 100% du pourcentage total de polymères et de charges) et pour chacun d'eux sont mesurées : la température de ramollissement (TR), la pénétration à 25°C (Pen 25°C) et 60°C (Pen 60°C), la viscosité Brookfield à 180°C (Visc 180°C) et la flexibilité à basse température, à l'état neuf (FBT) et à l'état vieilli ($FBT_4$).

Le pourcentage de polymères dans le bitume modifié est généralement compris entre environ 19 et 26 % en poids, le reste étant composé de bitume et de charges. Dans cette gamme, le mélange se présente sous la forme d'une phase polymère continue dans laquelle le bitume est dispersé.

Des études préalables ayant montré que beaucoup d'interactions pouvaient être négligées, le nombre de mélanges se réduit à 16.

On réalise 16 mélanges, chaque mélange comprenant un pourcentage minimum ou maximum de chacun des 6 polymères, correspondant à un niveau maximum et à un niveau minimum d'une gamme de valeurs déterminée à l'avance pour chaque polymère.

Par exemple, un de ces mélanges comprendra un niveau maximum d'IPP, d'APP2 et de COPO1 et un niveau minimum d'APP1, de COPO2 et COPO3.

Egalement à titre d'exemple, les gammes de valeurs de chaque polymère sont les suivantes (% en poids) :

| IPP | 2,5 - 3,5% | COPO1 | 1,7 - 2,8% |
|------|------------|-------|------------|
| APP1 | 6,3 - 8,7% | COPO2 | 1,3 - 2,4% |
| APP2 | 4.8 - 6.2 % | COPO3 | 1,7 - 2.8% |

Des mélanges complémentaires ont également été réalisés, en utilisant le pourcentage moyen de chaque polymère, pour vérifier la linéarité des modèles et contrôler la reproductibilité des méthodes de mesure. Au total, 20 mélanges sont ainsi effectués.

Les évaluations statistiques peuvent être effectuées au moyen d'un logiciel du type Stat Graphics Plus 1.1.

A l'aide de cet outil informatique, les résultats des mesures obtenus pour chacune des caractéristiques physiques et pour chacun des 20 mélanges bitume/polymères, permettent de modéliser facilement les différentes caractéristiques physiques.

Dans l'exemple ici décrit, les modèles obtenus sont les suivants en utilisant une notation normalisée en -1 et +1 des variables :

$$(1) \quad TR : 153{,}1 + 0{,}92 \cdot IPP + 0{,}33 \cdot APP1 + 0{,}24 \cdot COPO3$$
$$+ 0{,}22 \cdot IPP \cdot COPO2$$

$$(2) \quad Pen_{25°C} : 35 - 2{,}13 \cdot IPP - 0{,}56 \cdot APP1$$

$$(3) \quad Pen_{60°C} : 147 - 19{,}5 \cdot IPP - 5{,}9 \cdot APP1 -$$
$$0{,}14 \cdot IPP \cdot APP1$$

$$(4) \quad Ln(Visc_{180°C}) : 7{,}89 + 0{,}087 \cdot IPP + 0{,}028 \cdot APP1 +$$
$$0{,}13 \cdot COPO1 + 0{,}096 \cdot COPO2 + 0{,}083 \cdot COPO3$$
$$+$$
$$0{,}011 \cdot IPP \cdot APP1 + 0{,}011 \cdot IPP \cdot COPO2$$

$$(5) \quad FBT : -15 + 0{,}69 \cdot IPP - 0{,}69 \cdot APP2 - 0{,}81 \cdot COPO1$$

$$(6) \quad FBT4 : -10{,}2 + 1{,}44 \cdot IPP - 0{,}94 \cdot APP2 - 0{,}94 \cdot COPO1$$
$$- 1{,}19 \cdot COPO2$$

La constante qui apparaît dans chaque modèle donne une estimation de la caractéristique physique correspondante du mélange, pour une formulation de polymères comprenant des valeurs moyennes.

Lorsque l'effet d'un polymère sur la constante en tête des modèles (1) à (6) est négligeable, le polymère n'apparaît pas dans le modèle. Par ailleurs, la méthode utilisée permet de mettre en évidence d'éventuelles interactions entre les polymères.

Ces modèles font apparaître que la température de ramollissement et la pénétration à 25 et 60°C, dépendent essentiellement de la quantité d'IPP, alors que la viscosité et la flexibilité sont influencées par pratiquement tous les polymères, le pourcentage de chaque polymère variant dans les gammes précédemment indiquées.

Pour un exemple de mélange, vont être indiquées les valeurs des caractéristiques physiques prédites par les modèles et les valeurs mesurées de ces mêmes caractéristiques physiques.

Les pourcentages en poids des polymères dans l'exemple retenu (déjà indiqué précédemment) sont les suivants :

| | |
|---|---|
| IPP : 3,5 % | COPO1 : 2,8 % |
| APP1 : 6,3 % | COPO2 : 1,3 % |
| APP2 : 6,2 % | COPO3 : 1,7 % |

Pour cet exemple, les valeurs des caractéristiques physiques, données par les modèles (1) à (6) sont les suivantes :

| | |
|---|---|
| TR: 153,2 °C | $Visc_{180°C}$ : 2,64 Pa·s (2 640 CP) |
| $Pen_{25°C}$ : 33,4 decimm | FBT : -15,8 °C |
| $Pen_{60°C}$ : 133 decimm | $FBT_4$ : -9,5 °C |

Les valeurs mesurées de ces mêmes caractéristiques physiques sont :

| | |
|---|---|
| TR: 153,5 °C | $Visc_{180°C}$ : 2,68 Pa·s (2 680 CP) |
| $Pen_{25°C}$ : 33 decimm | FBT : -15 °C |
| $Pen_{60°C}$ : 131 decimm | $FBT_4$ : -9 °C |

Cet exemple montre que les modèles (1) à (6) sont fiables. Ceci a également été confirmé par les nombreux essais réalisés.

Les modèles, tels que (1) à (6) précédents, présentent par ailleurs un autre avantage considérable.

Ils peuvent facilement être modifiés pour tenir compte d'un autre polymère. Par exemple, si un des copolymères qui ont été utilisés pour définir le modèle n'est plus en stock et se trouve remplacé par un nouveau copolymère, ou si un nouveau lot d'un des APP est reçu par le fabricant, les modèles précédemment mis au point ne sont pas directement utilisables. Cependant, il n'est pas nécessaire de remettre en oeuvre un plan d'expériences et de refaire l'ensemble des mesures, pour toutes les caractéristiques physiques, afin d'établir de nouveaux modèles.

Pour déterminer l'influence du nouveau constituant, il a été établi qu'un nombre limité de mélanges, au minimum 4, et des mesures des caractéristiques physiques à modéliser étaient suffisants.

La composition des mélanges avec le nouveau constituant sont choisies comme lors de l'élaboration du modèle initial, en retenant un pourcentage maximum, un pourcentage minimum et un pourcentage intermédiaire.

Les résultats obtenus permettent de modifier de façon simple, rapide et peu coûteuse le modèle établi initialement pour intégrer le nouveau polymère.

Les modifications que l'on peut apporter ne sont pas limitées à l'introduction d'un seul polymère dans les modèles.

Le fabricant dispose donc très facilement de modèles adaptés aux produits qu'il détient effectivement en stock.

Par exemple, si l'un des polymères n'est plus en stock, le modèle reste valable, il suffit d'adapter les quantités des autres polymères.

Les modèles permettent aussi de déterminer les caractéristiques physiques d'un mélange donné, en fonction des pourcentages de polymères utilisés. Le fabricant peut alors retenir ou non le mélange, selon que ces caractéristiques entrent ou non dans les spécifications techniques auxquelles il doit se conformer sans avoir besoin de réaliser un mélange spécifique.

Il peut également déterminer rapidement comment les quantités de chaque polymère doivent être modifiées pour respecter les spécifications techniques.

Ceci constitue un avantage considérable par rapport aux méthodes classiques aujourd'hui utilisées et qui sont essentiellement empiriques : à chaque fois que l'on veut modifier la quantité d'un polymère (par exemple : ajout d'un nouveau polymère, ajustement d'une caractéristique physique du mélange, rupture de stock d'un polymère), le fabricant procède par tâtonnements en réalisant au moins un ou deux mélanges dans son laboratoire pour vérifier que les caractéristiques physiques du mélange respectent bien les spécifications techniques. La fréquence de ces modifications peut être supérieure à une par jour.

Enfin, ces méthodes empiriques sont relativement peu fiables. C'est pour cette raison que les fabricants ont tendance à surdoser les polymères présents dans le mélange. Or, ce surdosage grève le coût des mélanges et donc des membranes d'étanchéité.

Le fabricant peut également utiliser les modèles pour optimiser la formulation du mélange, en déterminant les pourcentages de chaque polymère en fonction de caractéristiques physiques de valeur donnée et en intégrant éventuellement des questions de coût, ou des paramètres de manufacturabilité (par exemple la nature des armatures de renforcement utilisées).

Ainsi, le fabricant n'a besoin d'effectuer qu'un nombre limité de tests une fois pour toutes. A partir des modèles établis, il peut établir de nouvelles formulations du mélange, pour un bitume donné, répondant à des spécifications particulières. Aucun autre test n'est nécessaire.

Ceci constitue un avantage par rapport aux méthodes empiriques classiques qui demandent systématiquement de nouveaux essais, lorsque les spécifications concernant les caractéristiques physiques du mélange sont modifiées.

L'invention comporte également des avantages lors des contrôles en cours de fabrication.

Les fabricants effectuent classiquement ces contrôles, notamment en réalisant des prélèvements qui sont ensuite analysés. Si les tests montrent que le mélange bitume/polymères obtenu ne respecte pas les spécifications techniques, il faut d'une part, modifier la formulation du mélange en amont et d'autre part, corriger le mélange déjà obtenu pour qu'il réponde aux spécifications. Cette méthode est longue et coûteuse à mettre en oeuvre et comporte de nombreux aléas.

L'invention permet tout d'abord de corriger les éventuels écarts en fabrication par rapport aux spécifications techniques de façon simple.

Des prélèvements sont effectués sur le mélange fabriqué.

Des mesures des caractéristiques physiques du mélange sont effectuées et comparées avec les valeurs théoriques attendues par l'utilisation des modèles ou les spécifications techniques.

Si un écart notable est constaté, le fabricant effectue des investigations. Elles mettent, par exemple, en évidence une erreur dans le chargement du bitume ou d'un des polymères. Cette erreur est donc corrigée immédiatement.

Elle permet également de facilement corriger le mélange déjà obtenu, cette correction étant obtenue en ajoutant des constituants. Là encore, les modèles précédemment établis servent à déterminer facilement la correction à apporter, puisqu'ils permettent d'anticiper l'effet d'un ajout d'un constituant.

A titre d'exemple, si le mélange obtenu n'est pas assez visqueux, le modèle (4) permet de facilement prédire l'effet d'un changement de quantité d'un polymère, par exemple l'IPP ou le COPO2.

Le fabricant peut ainsi aboutir, sans tests complémentaires, à plusieurs solutions de correction du mélange. Il effectuera alors un choix, notamment sur des critères de coûts.

Il convient de noter que les caractéristiques physiques d'un mélange sont modélisées pour un bitume déterminé. La comparaison des modèles établis pour plusieurs bitumes donne également au fabricant des indications sur le choix du bitume, en fonction des polymères utilisés et des caractéristiques physiques souhaitées.

Ainsi, l'invention permet au fabricant de mélanges bitume/polymères de prévoir les effets d'un changement de la composition des polymères sur les caractéristiques physiques du mélange. En connaissant à l'avance l'influence de chaque polymère, pour un bitume donné, il peut adapter la composition de polymères, sans que des tests complémentaires soient nécessaires et il peut respecter les spécifications techniques qui lui sont imposées.

**Revendications**

1. Procédé de détermination de l'influence du pourcentage des différents polymères d'un mélange bitume/polymère sur au moins une caractéristique physique dudit mélange, consistant :

   • à réaliser un nombre limité de mélanges bitume/polymères, selon la méthode des plans d'expériences, les polymères et le bitume étant identiques dans tous les mélanges,
   • à mesurer la(les) dite(s) caractéristique(s) physique(s) pour chacun desdits mélanges,
   • à en déduire, par des calculs statistiques, une modélisation de la(des) dite(s) caractéristique(s) physique(s), en fonction du pourcentage des différents polymères,

   **caractérisé en ce qu'**il consiste en outre

- à réaliser ledit nombre limité de mélanges bitume/polymères avec des pourcentages respectifs des polymères fixés à un niveau maximum, ou à un niveau minimum et à un niveau intermédiaire d'une gamme de valeurs déterminée pour chaque polymère,
- à réaliser au moins quatre mélanges supplémentaires incorporant un autre polymère en retenant un pourcentage maximum, un pourcentage minimum et un pourcentage intermédiaire,
- à mesurer la(les) dite(s) caractéristique(s) physique(s) pour chacun de ces mélanges supplémentaires,
- et à en déduire les modifications à apporter à la modélisation de la (des) dite(s) caractéristique(s) physique(s), pour tenir compte de l'introduction de cet autre polymère.

2. Procédé selon la revendication 1, le mélange bitume/polymère comportant des charges.

3. Procédé selon la revendication 2, le pourcentage de charges étant constant dans le mélange.

4. Procédé selon l'une des revendications 1 à 3, la(les) caractéristiques(s) physique(s) du mélange étant la flexibilité à basse température à l'état neuf et à l'état vieilli, la viscosité Brookfield à 180°C, la pénétration à 25°C et 60°C et/ou la température de ramollissement.

5. Procédé selon l'une des revendications 1 à 4, les polymères dudit mélange bitume/polymères étant choisis parmi les APP, IPP et les copolymères du type éthylène/propylène.

6. Procédé de détermination d'une(de) caractéristique(s) physique(s) d'un mélange bitume/polymères, en fonction des polymères utilisés consistant,

   - à mettre en oeuvre le procédé selon l'une des revendications 1 à 5 pour établir l'influence du pourcentage de chaque polymère sur la (les)dite(s) caractéristique(s) physique(s) du mélange,
   - et à fixer les pourcentages de chaque polymère pour en déduire la valeur de la(des) dite(s) caractéristique(s) physique(s).

7. Procédé d'obtention d'un mélange bitume/polymères présentant une (des) caractéristique(s) physique(s) déterminée(s) consistant :

   - à mettre en oeuvre le procédé selon l'une des revendications 1 à 5 pour établir l'influence du pourcentage de chaque polymère sur la(les)dite(s) caractéristique(s) physique(s),
   - à fixer une gamme de valeurs pour la(les)dite(s) caractéristique(s), et
   - à déduire le pourcentage de chaque polymère dans le mélange.

8. Procédé selon la revendication 7, consistant à déterminer les pourcentages des polymères en tenant également compte de leur coût.

9. Procédé de modification d'un mélange bitume/polymères présentant une(des) caractéristique(s) physique(s) de valeur déterminée,

   - à mettre en oeuvre le procédé selon l'une des revendications 1 à 5 pour établir l'influence du pourcentage de chaque polymère dans le mélange, sur la(les)dite(s) caractéristique(s) physique(s),
   - à fixer d'autres valeurs de la(des)dite(s) caractéristique(s) physique(s) dudit mélange, et
   - à calculer la quantité supplémentaire d'au moins un polymère à ajouter dans ledit mélange pour qu'il présente des caractéristiques physiques de valeurs sensiblement égale à ces autres valeurs.

**Patentansprüche**

1. Verfahren zur Bestimmung des Einflusses des Prozentsatzes von verschiedenen Polymeren eines Bitumen/Polymer-Gemisches auf mindestens eine physikalische Eigenschaft des Gemisches, bestehend

   - in dem Herstellen einer begrenzten Anzahl von Bitumen/Polymer-Gemischen nach der Methode der Erfahrungsschemata, wobei die Polymere une das Bitumen in allen Gemischen identisch sind und die jeweiligen Prozentsätze der Polymere auf einem maximalen oder minimalen Niveau einer bestimmten Werteskala für jedes Polymer festgesetzt werden können,

- in dem Messen der erwähnten physikalischen Eigenschaft(en) für jedes der Gemische, und
- in dem Ableiten einer Modellierung der physikalischen Eigenschaft(en) durch statistische Berechnungen in Abhängigkeit von Prozentsatz der verschiedenen Polymere, **dadurch gekennzeichnet, dass** es ferner
- in dem Herstellen der begrenzten Anzahl von Bitumen/Polymer-Gemischen mit jeweiligen Prozentsätzen der Polymere, die auf einem obersten Wert oder auf einem untersten Wert und einem Zwischenwert einer für jedes Polymer bestimmten Reihe von Werten gestgelegt sind,
- in dem Herstellen von mindestens vier zusätzlichen Gemischen, die ein weiteres Polymer enthalten, in Abhänggigkeit von den Prozentsätzen der verscheidenen Polymere,
- in dem Messen der physikalischen Eigenschaft(en) jedes dieser zusätzlichen Gemische, und
- in dem Ableiten der an der Modellierung der physikalischen Eigenschaft(en) vorzunehmenden Änderungen, um die Einführung dieses weiteren Polymers zu berücksichtigen.

2. Verfahren nach Anspruch 1, bei dem das Bitumen/Polymer-Gemisch Füllstoffe umfaßt.

3. Verfahren nach Anspruch 2, bei dem der Prozentsatz von Füllstoffen in dem Gemisch konstant ist.

4. Verfahren nach einem der Ansprüche 1 bis 3, bei dem die Eigenschaft(en) des Gemisches die Niedertemperaturflexibilität im neuen und im alten Zustand, die Brookfield-Viskosität bei 180°C, die Penetration bei 25°C und 60°C und/oder die Erweichungstemperatur ist (sind).

5. Verfahren nach einem der Ansprüche 1 bis 4, bei dem die Polymere des Bitumen/Polymer-Gemisches unter den APP, IPP und den Copolymeren vom Typ Äthylen/Propylen ausgewählt werden.

6. Verfahren zur Bestimmung einer (von) physikalischen Eigenschaft(en) eines Bitumen/Polymer-Gemisches in Abhängigkeit von den verwendeten Polymeren, bestehend

- in dem Durchführen des Verfahrens nach einem der Ansprüche 1 bis 5, um den Einfluß des Prozentsatzes jedes Polymers auf die physikalische(n) Eigenschaft(en) des Gemisches festzustellen, und
- in dem Festlegen der Prozentsätze jedes Polymers, um davon den Wert der physikalischen Eigenschaft(en) abzuleiten.

7. Verfahren zur Gewinnung eines Bitumen/Polymer-Gemisches, das eine bestimmte oder bestimmte physikalische Eigenschaft(en) aufweist, bestehend

- in dem Durchführen des Verfahrens nach einem der Ansprüche 1 bis 5, um den Einfluß des Prozentsatzes jedes Polymers auf die physikalische(n) Eigenschaft(en) festzustellen,
- in dem Festlegen einer Werteskala für die Eigenschaft(en), und
- in dem Ableiten des Prozentsatzes jedes Polymers in dem Gemisch.

8. Verfahren nach Anspruch 7, bestehend in dem Bestimmen der Prozentsätze der Polymere, indem auch ihre Kosten berücksichtigt werden.

9. Verfahren zur Änderung eines Bitumen/Polymer-Gemisches, das eine physikalische Eigenschaft(en) mit einem bestimmten Wert aufweist, bestehend

- in dem Durchführen des Verfahrens nach einem der Ansprüche 1 bis 5, um den Einfluß des Prozentsatzes jedes Polymers in dem Gemisch auf die physikalische(n) Eigenschaft(en) zu ermitteln,
- in dem Festlegen weiterer Werte der physikalischen Eigenschaft(en) des Gemisches, und
- in dem Berechnen der zusätzlichen Menge mindestens eines dem Gemisch hinzuzusetzenden Polymers, damit das Gemisch physikalische Eigenschaften mit Werten aufweist, die im wesentlichen gleich diesen anderen Werten sind.

**Claims**

1. Method for determining the influence of the percentage of the various polymers of a bitumen/polymers mixture on at least one physical characteristic of the said mixture, consisting:

- in preparing a limited number of bitumen/polymers mixtures according to the method of experimental designs, the polymers and the bitumen being identical in all the mixtures and it being possible for the respective percentages of the polymers to be set at a maximum level or at a minimum level of a predetermined range of values for each polymer,
- in measuring the said physical characteristic(s) for each of the said mixtures,
- and in deducing therefrom, by statistical calculations, a model of the said physical characteristic(s), as a function of the respective percentages of the polymers,

    **characterized by** consisting additionally:

- in preparing the limited number of bitumen/polymers mixtures with the respective percentages of polymers being fixed at a maximum level or at a minimum level and an intermediate level of a range of values determined for each polymer,
- in preparing at least four additional mixtures incorporating another polymer with respectively a maximum percentage, a minimum percentage and an intermediate percentage,
- in measuring the said physical characteristic(s) for each of these additional mixtures,
- and in deducing therefrom the modifications to be introduced into the model of the said physical characteristic(s) in order to take into account the introduction of this other polymer.

2. Method according to Claim 1, the bitumen/polymers mixture comprising fillers.

3. Method according to Claim 3, the percentage of fillers being constant in the mixture.

4. Method according to one of Claims 1 to 3, the physical characteristic(s) of the mixture being the flexibility at low temperature in the fresh state and in the aged state, the Brookfield viscosity at 180°C, the penetration at 25°C and 60°C and/or the softening temperature.

5. Method according to one of Claims 1 to 4, the polymers of the said bitumen/polymers mixture being chosen from APP, IPP and copolymers of the ethylene/propylene type.

6. Method of determining (a) physical characteristic(s) of a bitumen/polymers mixture as a function of the polymers used, consisting,

- in employing the method according to one of Claims 1 to 5 in order to establish the influence of the percentage of each polymer on the said physical characteristic(s) of the mixture,
- and in setting the percentages of each polymer in order to deduce therefrom the value of the said physical characteristic(s).

7. Method of producing a bitumen/polymers mixture exhibiting (a) predetermined physical characteristic(s), consisting :

- in employing the method according to one of Claims 1 to 5 in order to establish the influence of the percentage of each polymer on the said physical characteristic(s),
- in setting a range of values for the said characteristic(s) and
- in deducing the percentage of each polymer in the mixture.

8. Method according to Claim 7, consisting in determining the percentages of the polymers while also taking into account their cost.

9. Method for modifying a bitumen/polymers mixture exhibiting (a) physical characteristic(s) of predetermined value, consisting

- in employing the method according to one of Claims 1 to 5 in order to establish the influence of the percentage of each polymer in the mixture on the said physical characteristic(s),
- in setting other values of the said physical characteristic(s) of the said mixture and
- in calculating the additional amount of at least one polymer to be added to the said mixture in order for it to exhibit physical characteristics with values substantially equal to these other values.